# EUROPEAN PATENT APPLICATION

(11) **EP 3 255 413 A1**
(43) Date of publication of application: **13.12.2017**
(21) Application number: 16746527.7
(22) Date of filing: 29.01.2016
(51) Int. Cl.: G01N 21/41

(54) **MEASUREMENT DEVICE**

(30) Priority: 05.02.2015 JP 2015020936
(71) Applicant: Nitto Denko Corporation, Ibaraki-shi, Osaka 567-8680 (JP)
(72) Inventor: IHARA, Terukazu, Ibaraki-shi Osaka 567-8680 (JP); NISHIO, Hajime, Ibaraki-shi Osaka 567-8680 (JP); HATAKEYAMA, Yoshiharu, Ibaraki-shi Osaka 567-8680 (JP); YANO, Chiharu, Ibaraki-shi Osaka 567-8680 (JP); NAKANO, Junko, Ibaraki-shi Osaka 567-8680 (JP)
(74) Representative: Bird & Bird LLP
(86) International application number: PCT/JP2016/052646
(87) International publication number: WO 2016/125696

(57) **Abstract**

The present invention provides a measurement device which is compact and portable and is simply operated, and which is capable of quantifying the amount of an object to be inspected with high sensitivity and high accuracy. A measurement device of the present invention includes an SPR sensor cell, a light source, and a light receiving element. The SPR sensor cell includes a plurality of optical waveguides. At least one of the plurality of optical waveguides forms a detection unit together with a metal layer onto which a recognition substance for an object to be inspected is fixed, and at least another one of the plurality of optical waveguides forms a reference unit together with a metal layer free of the recognition substance.

## Description

### Technical Field

The present invention relates to a measurement device, and more specifically, to a measurement device configured to measure an amount of an object to be inspected through use of surface plasmon resonance and an optical waveguide.

### Background Art

A measurement device configured to measure the amount of an obj ect to be inspected through use of a surface plasmon resonance (SPR) phenomenon has been known. Such measurement device can be used for various chemical analyses and biochemical analyses, for example, measurement of the concentration of a sample and detection of an immune reaction. As an example of the application of those chemical analyses and biochemical analyses, there are given detection of an allergen and/or quantification of the amount of the allergen. In particular, a main allergen in allergy diseases such as infant asthma and atopic dermatitis is considered to be derived frommites contained in indoor dust (house dust). Therefore, it is significantly important for such allergy patients to quantify the amount of the mite allergen in an environment.

As a device configured to measure the amount of the mite allergen through use of the SPR phenomenon, there has been proposed a mite allergen measurement device using prism-type SPR, which is configured to measure a resonance angle (Patent Literature 1). However, in the measurement device disclosed in Patent Literature 1, it is necessary to measure a significantly minute resonance angle change (0.0072° to 0.0000288°), and as a result, a large and precise drive device is required. Therefore, the measurement device disclosed in Patent Literature 1 is used under the premise of being installed in a research facility having a measurement environment controlled precisely. In other words, the measurement device disclosed in Patent Literature 1 is not suitable for simply measuring the amount of the mite allergen in an ordinary environment (for example, a hot and humid state filled with dust) as in a general house and is not assumed to be portable. Further, in the measurement device disclosed in Patent Literature 1, in order to set a sample case (sensor chip), it is necessary to apply matching oil between a prism and the sample case, and hence an operation is significantly complicated. In addition, the amount of the allergen extracted from house dust is extremely small, and the aqueous solution amount thereof is also small. Therefore, in a prism-type measurement device using only one reflection, the sensitivity is liable to be insufficient, and hence high accuracy is required in the fixing amount of an anti-allergen antibody, and a light source and a light receiving element.

The measurement device for the amount of the mite allergen has been described as a typical example of the measurement device using the SPR phenomenon. However, the above-mentioned problems are not limited to the quantification of the amount of the mite allergen and are common to a measurement device configured to measure and/or quantify the amount of an object to be inspected through use of the SPR phenomenon.

### Citation List

### Patent Literature

[PTL 1] JP 2002-148180 A

### Summary of Invention

### Technical Problem

The present invention has been made to solve the above-mentioned problems, and an object of the present invention is to provide a measurement device which is compact and portable and is simply operated, and which is capable of quantifying the amount of an object to be inspected with high sensitivity and high accuracy.

### Solution to Problem

A measurement device according to one embodiment of the present invention includes an SPR sensor cell, a light source, and a light receiving element. The SPR sensor cell includes a plurality of optical waveguides, at least one of the plurality of optical waveguides forms a detection unit together with a metal layer onto which a recognition substance for an object to be inspected is fixed, and at least another one of the plurality of optical waveguides forms a reference unit together with a metal layer free of the recognition substance.

In one embodiment, the measurement device further includes a development unit extending across the plurality of optical waveguides. In one embodiment, the development unit includes a porous material.

In one embodiment, the detection unit includes an under-cladding layer, a core layer formed so that at least a part thereof is adjacent to the under-cladding layer, and a metal layer configured to cover the core layer, and the recognition substance is fixed onto the metal layer.

In one embodiment, the SPR sensor cell is removably mounted.

In one embodiment, the recognition substance includes an anti-allergen antibody, and the measurement device is configured to measure an amount of an allergen by an antigen-antibody reaction with the anti-allergen antibody.

### Advantageous Effects of Invention

According to the embodiment of the present invention, in the measurement device using the SPR phenomenon, the SPR sensor cell includes the plurality of optical waveguides. At least one of the optical waveguides is used as the detection unit onto which the recognition substance for the object to be inspected is fixed, and at least another one of the optical waveguides is used as the reference unit free of the recognition substance. With this, it is possible to realize the measurement device which is compact and portable and is simply operated, and which is capable of quantifying the amount of the object to be inspected with high sensitivity and high accuracy. The measurement device according to the embodiment of the present invention can be easily used by a person (for example, a housewife) having no expertise in a home environment.

### Brief Description of Drawings

FIG. **1** is a schematic perspective view for illustrating a measurement device according to one embodiment of the present invention.
FIG. **2** is a schematic perspective view of main parts of the measurement device of FIG. **1** when viewed from above.
FIG. **3** is a schematic perspective view for illustrating an SPR sensor cell to be used in one embodiment of the present invention.
FIG. **4** is a schematic plan view of the SPR sensor cell of FIG. **3****.**
FIG. **5** is a schematic exploded perspective view of the SPR sensor cell of FIG. **3****.**
FIG. **6** is a schematic sectional view taken along the line **A1-A1** of an upper plate of the SPR sensor cell of FIG. **3****.**
FIG. **7** is a schematic sectional view taken along the line **A2-A2** of a lower plate of the SPR sensor cell of FIG. **3****.**
FIGS. **8** are each a schematic view for illustrating a sample liquid preparation and dropping member to be used at the time of use of the measurement device according to one embodiment of the present invention.
FIG. **9** is a graph for showing a transmittance before dropping of a sample liquid in a measurement device of Example 1.
FIG. **10** is a graph for showing the comparison of a transmittance change between a detection unit and a reference unit after dropping of the sample liquid in the measurement device of Example 1.
FIG. **11** is a graph for showing the difference in transmittance change between the detection unit and the reference unit after dropping of the sample liquid in the measurement device of Example 1.
FIG. **12** is a graph for showing the comparison of a transmittance change between a detection unit and a reference unit after dropping of a sample liquid in a measurement device of Example 2.
FIG. **13** is a graph for showing the difference in transmittance change between the detection unit and the reference unit after dropping of the sample liquid in the measurement device of Example 2.
FIG. **14** is a graph for showing a transmittance change in a detection unit after dropping of a sample liquid in a measurement device of Comparative Example 1.

### Description of Embodiments

### A. Measurement Device

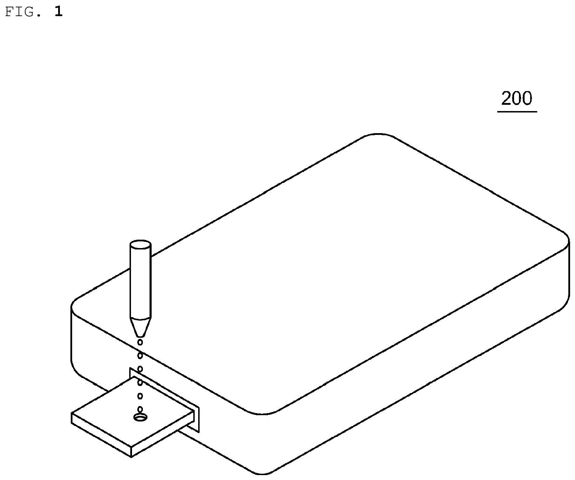

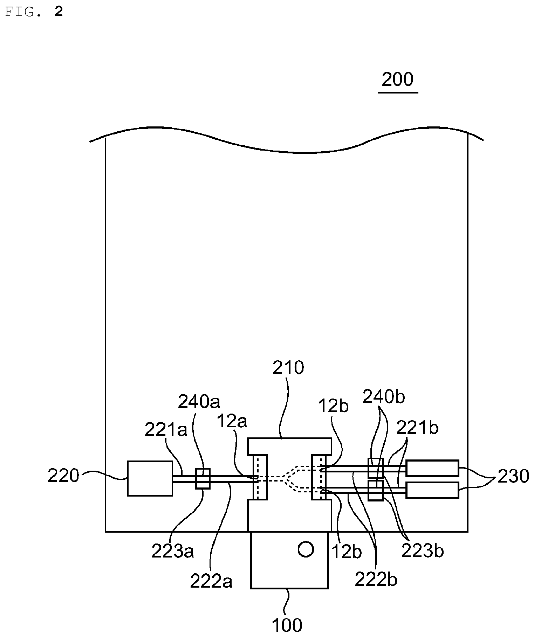

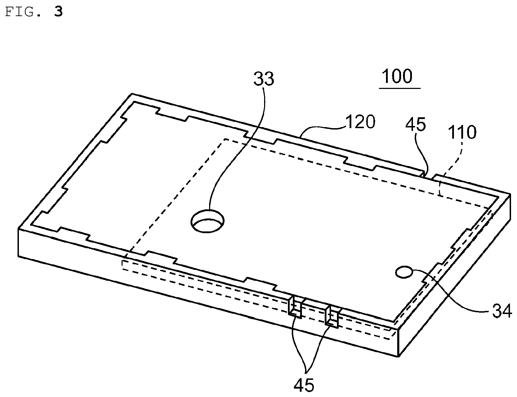

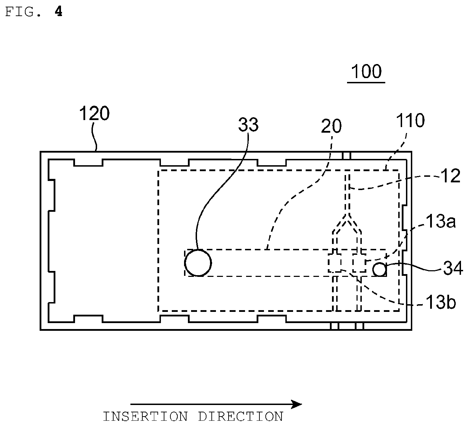

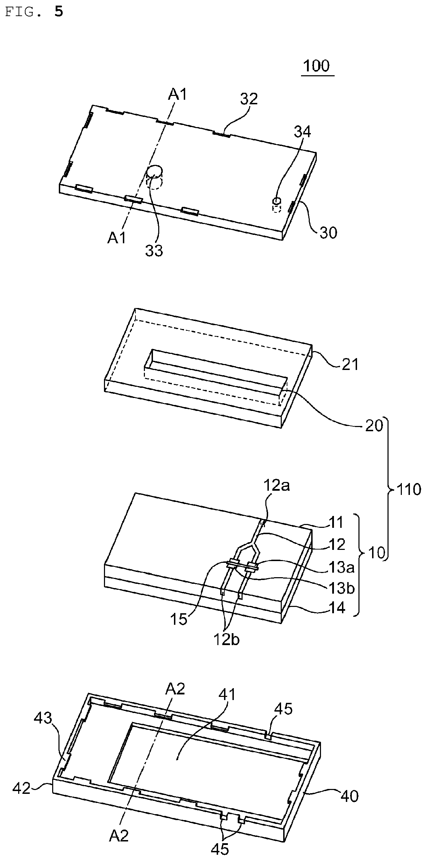

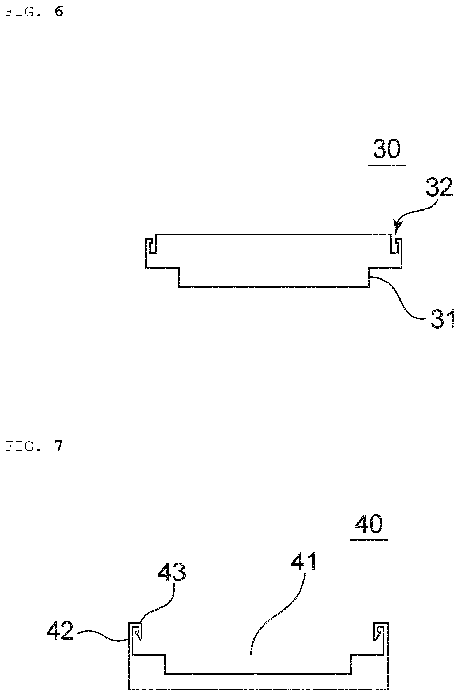

FIG. **1** is a schematic perspective view of a measurement device according to one embodiment of the present invention. FIG. **2** is a schematic perspective view of the measurement device of FIG. **1** when viewed from above. FIG. **3** is a schematic perspective view of an example of an SPR sensor cell to be used in the measurement device according to the embodiment of the present invention. FIG. **4** is a schematic plan view of the SPR sensor cell of FIG. **3** when viewed from above. FIG. **5** is a schematic exploded perspective view of the SPR sensor cell of FIG. **3****.** FIG. **6** is a schematic sectional view taken along the line **A1-A1** of an upper plate of the SPR sensor cell of FIG. **3****.** FIG. **7** is a schematic sectional view taken along the line **A2-A2** of a lower plate of the SPR sensor cell of FIG. **3****.** For ease of viewing, each constituent member in the drawings is schematically illustrated, and the size and/or scale thereof are different from the actual size and/or scale.

A measurement device **200** includes an SPR sensor cell **100,** a light source **220,** and light receiving elements (light measuring instruments) **230.** The SPR sensor cell **100** is typically removably mounted on a cell mounting portion **210** to be incorporated into the measurement device **200**. The SPR sensor cell **100** is typically inserted into the cell mounting portion **210** in a direction orthogonal to a waveguide direction of an optical waveguide (direction in which a core layer **12** extends) in plan view. In the cell mounting portion **210,** in one embodiment, when the SPR sensor cell **100** is mounted at a predetermined position of the cell mounting portion **210,** the light source **220,** a light source-side optical fiber **240a,** the optical waveguide (substantially, the core layer **12**) of the SPR sensor cell **100,** light receiving element-side optical fibers **240b,** and the light receiving elements **230** are arranged so as to be positioned on one axis or on an axis parallel to the one axis. More specifically, in the measurement device **200,** in one embodiment, as in the illustrated example, the light source **220,** the light source-side optical fiber **240a,** and the optical waveguide (substantially, the core layer **12**) of the SPR sensor cell **100** are arranged so as to be positioned uniaxially on the light source side, and two branched optical waveguides (substantially, the core layers **12**) of the SPR sensor cell **100** and the two light receiving elements **230** are arranged so as to be positioned uniaxially on the light receiving element side, respectively. With such configuration, the attenuation amount of light in the optical waveguides can be minimized, and hence a light amount sufficient for measurement of SPR can be obtained. As a result, the amount of an object to be inspected (for example, an allergen) can be measured with high sensitivity and high accuracy.

As the light source **220,** any suitable light source may be adopted. Specific examples of the light source include a white light source, a monochromatic light source, and a light-emitting diode. The light-emitting diode is preferred. Long lifetime, low cost, and elimination of maintenance can be realized through use of the light-emitting diode as the light source. The light receiving elements (light measuring instruments) **230** are connected to any suitable arithmetic processing device so as to enable accumulation, display, and processing of data. As each of the light receiving elements **230,** any suitable light receiving element may be adopted. A photodiode is preferred. Excellent light receiving sensitivity can be realized to enlarge a measurement range through use of the photodiode as the light receiving element.

The light source **220** is connected to the light source-side optical fiber **240a** through a light source-side optical connector **221a.** The light source-side optical fiber **240a** is connected to a light incidence port **12a** of the core layer **12** of the SPR sensor cell **100** through a light source-side fiber block **222a.** The light receiving element-side optical fibers **240b** are connected to light output ports **12b** of the core layers **12** through light receiving-element side fiber blocks **222b**. The light receiving element-side optical fibers **240b** are connected to the light receiving elements **230** through light receiving-element side optical connectors **221b.** The light source-side optical fiber **240a** is fixed with a light source-side optical fiber fixing device **223a,** and the light receiving element-side optical fibers **240b** are fixed with light receiving-element side optical fiber fixing devices **223b.**

The SPR sensor cell **100** includes an optical waveguide and is configured to detect the state of a sample and/or a change thereof by causing light having a specific wavelength in light introduced into the optical waveguide to generate surface plasmon resonance in a metal layer and attenuating the intensity of the light. In the embodiment of the present invention, the SPR sensor cell **100** includes a plurality of (two in the illustrated example) optical waveguides (substantially, the core layers **12** and **12**)**.** At least one of the plurality of optical waveguides forms a detection unit **13a** together with a metal layer onto which a recognition substance for the object to be inspected is fixed, and at least another one of the plurality of optical waveguides forms a reference unit **13b** together with a metal layer free of the recognition substance. With such configuration, a light source noise, an environmental noise, and the like can be determined based on a change in light intensity of the reference unit **13b.** Therefore, the amount of the object to be inspected can be measured (quantified) more precisely by correcting a change in light intensity of the detection unit **13a** with the change in light intensity of the reference unit **13b.** The accurate amount of the object to be inspected, having the light source noise, the environmental noise, and the like removed therefrom, can be measured, for example, by previously creating a reference line indicating a relative relationship between the difference of the light intensity in the reference unit **13b** and the light intensity in the detection unit **13a,** and the amount of the obj ect to be inspected. The recognition substance as used herein refers to a substance that exhibits a specific reaction, binding, adsorption, response, or the like with respect to the object to be inspected. The detailed configuration of the SPR sensor cell is described later in the section B.

The downsizing of the measurement device **200** is realized by adopting the above-mentioned configuration. Specifically, the light-emitting diode and the photodiode can be used as single elements, and the light amount and detection accuracy can also be maintained at sufficient levels. Further, through the above-mentioned uniaxial arrangement, the amount of light propagating through the optical waveguide can be ensured satisfactorily. Thus, both the downsizing and the measurement with high sensitivity and high accuracy can be satisfied. As a result, the measurement device (substantially, a measurement device main body: in which the SPR sensor cell is not mounted) can be installed in a home environment and is potable in one embodiment. The measurement device main body may have a vertical size of, for example, from about 50 mm to about 300 mm, a horizontal size of, for example, from about 50 mm to 250 mm, and a height of, for example, from about 20 mm to about 80 mm.

The measurement device **200** can measure (quantify) the amount of any suitable object to be inspected as long as the SPR phenomenon can be used. As a specific example of the object to be inspected, there is given an allergen (recognition substance: anti-allergen antibody that causes an antigen-antibody reaction with the allergen). In one embodiment, the measurement device **200** is used for quantifying an allergen. In this case, an anti-allergen antibody is fixed (typically, carried) onto the detection unit **13a** of the SPR sensor cell **100.** As a typical example of the allergen, there is given a mite allergen. When the measurement device **200** is used for quantifying a mite allergen, an anti-mite allergen antibody may be carried onto the detection unit **13a.**

### B. SPR Sensor Cell

### B-1. Entire Configuration of SPR Sensor Cell

The SPR sensor cell **100** includes an optical waveguide main body **10** and a sample mounting portion **20** (hereinafter, the optical waveguide main body **10** and the sample mounting portion **20** are sometimes collectively referred to as "cell main body **110"**). For practical purposes, the SPR sensor cell **100** further includes a holder **120** configured to hold and fix the cell main body **110.**

Through adoption of the optical waveguide, the SPR sensor cell **100** is downsized to be portable while maintaining the quantification of the amount of the object to be inspected (for example, an allergen) with high sensitivity and high accuracy. The specific description is given below. In a related-art SPR sensor, when light is caused to enter a detection site, it is necessary to cause light to enter the detection site at an appropriate angle. Therefore, an alignment mechanism unit becomes complicated (for example, automatic angle control of incident light with a motor or the like) to require a space, and as a result, it is inevitable that the SPR sensor be enlarged. Meanwhile, in the case of the optical waveguide, propagating light is naturally converged within a desired angle range without considering an incident angle by appropriately designing a refractive index difference between a core layer and a cladding layer forming the optical waveguide. Further, even when light is caused to enter two or more sites, such as the detection unit and the reference unit, as in the embodiment of the present invention, it is only necessary that the optical waveguide (substantially, the core layer) be branched, and the light may be caused to enter only one place. Therefore, the enlargement in size can be avoided. Thus, the SPR sensor cell **100** may have a vertical size of, for example, from about 30 mm to about 100 mm, a horizontal size of, for example, from about 15 mm to about 50 mm, and a thickness of, for example, from about 0.5 mm to about 10 mm. As a result, a person (for example, a housewife) having no expertise can use the SPR sensor cell **100** easily (that is, without requiring a skill) in a home environment.

### B-2. Optical Waveguide Main Body

### B-2-1. Entire Configuration of Optical Waveguide Main Body

The optical waveguide main body **10** includes an under-cladding layer **11,** a core layer **12** formed so that at least a part thereof is adj acent to the under-cladding layer **11**, a metal layer **13** configured to cover a part of the under-cladding layer and a part of the core layer, and a substrate **14** arranged on a bottom surface side of the under-cladding layer. In the illustrated example, the core layer **12** is buried in the under-cladding layer **11** so that an upper surface thereof is exposed from the under-cladding layer **11.** Light having a specific wavelength in light introduced into the core layer **12** causes surface plasmon resonance in the metal layer **13.** Based on the attenuation of the intensity of the light caused by a sample on the metal layer (sample including the object to be inspected) and a change in state of the sample, the state of the sample and/or a change therein on the surface of the metal layer can be detected.

As described above, according to the embodiment of the present invention, the SPR sensor cell **100** includes the plurality of optical waveguides. Specifically, the plurality of core layers **12, 12,** ··· are formed so as to be surrounded by the under-cladding layer **11** to provide the plurality of optical waveguides, and light propagates through the core layer portion of each of the optical waveguides. In the illustrated example, the two branched core layers **12** and **12** are formed so as to be buried in the under-cladding layer **11** to provide the two optical waveguides. One of the optical waveguides forms the detection unit **13a** together with the metal layer **13** which is formed so as to cover a part of the core layer **12** and onto which the recognition substance for the object to be inspected is fixed, and another one of the optical waveguides forms the reference unit **13b** together with the metal layer **13** which is formed so as to cover a part of the core layer **12** and onto which the recognition material is not fixed. As described above, the recognition substance refers to a substance that exhibits a specific reaction, binding, adsorption, response, or the like with respect to the object to be inspected. When the object to be inspected is, for example, an allergen, the recognition substance is an anti-allergen antibody that causes an antigen-antibody reaction with the allergen. When the object to be inspected is the allergen, in the detection unit **13a,** the allergen in a measurement sample is brought into contact with the anti-allergen antibody fixed onto the metal layer to cause an antigen-antibody reaction, and the amount of the allergen in the sample can be quantified based on a change (typically, attenuation) in light intensity caused by the antigen-antibody reaction. Meanwhile, in the reference unit **13a,** as described above, a light source noise, an environmental noise, and the like can be determined based on a change in light intensity. Therefore, the amount of the allergen can be quantified more precisely by correcting the change in light intensity of the detection unit **13a** with a change in light intensity of the reference unit **13b.** The accurate amount of the allergen, having the light source noise, the environmental noise, and the like removed therefrom, can be measured, for example, by previously creating a reference line indicating a relative relationship between the difference of the light intensity in the reference unit **13b** and the light intensity in the detection unit **13a,** and the amount of the allergen. In the illustrated example, the embodiment in which the core layer is branched into two layers is illustrated. However, for example, a plurality of core layers extending substantially in parallel to each other may be arranged side by side, or branched core layers and core layers arranged side by side may be combined.

In the illustrated example, description is given of the embodiment in which the two optical waveguides (substantially, the core layers) are arranged. However, three or more (for example, 3 to 8, preferably 3 to 6, more preferably 3 to 5) optical waveguides may be arranged. In this case, a plurality of detection units and/or a plurality of reference units may be used. For example, when five optical waveguides are arranged, four detection units and one reference unit, three detection units and two reference units, two detection units and three reference units, or one detection unit and four reference units may be used. For example, when four detection units and one reference unit are used, the recognition substances to be fixed onto the detection units may be the same in all the four detection units, in the three detection units, or in the two detection units, or may be different from each other in all the four detection units. For example, when five optical waveguides are arranged to measure (quantify) the amounts of mite allergens, the amounts of Derf I, Derf II, Derp I, and Derp II can be all quantified precisely by arranging one optical waveguide as the reference unit and fixing antibodies against Derf I, Derf II, Derp I, and Derp II onto four optical waveguides (detection units) . As a result, a relationship between the amount of each of the mite allergens in house dust, and the threshold value of sensitization and/or the threshold value of induction of an attack of asthma can be determined.

As necessary, a protective layer (not shown) may be formed on each upper surface of the under-cladding layer **11** and the core layer **12.** The protective layer is typically formed so as to cover the entire upper surfaces of the under-cladding layer **11** and the core layer **12.**

In one embodiment, a development unit **15** extending across the plurality of optical waveguides (more specifically, the detection unit and the reference unit) is arranged as in the illustrated example.

### B-2-2. Under-cladding Layer

The under-cladding layer **11** is formed into a shape of a plate having a substantially rectangular shape in plan view, with a predetermined thickness. The thickness of the under-cladding layer (thickness from an upper surface of the core layer) is, for example, from 5 µm to 400 µm.

The under-cladding layer **11** may be formed of any suitable material having a lower refractive index than the core layer described later. Specific examples thereof include a fluorine resin, an epoxy resin, a polyimide resin, a polyamide resin, a silicone resin, an acrylic resin, and modified products thereof (for example, a fluorene-modified product, a deuterium-modified product, and a fluorine-modified product in the case of the resins other than the fluorine resin). Those resins may be used alone or in combination thereof. Those resins can each be used as a photosensitive material preferably by being blended with a photosensitizing agent.

The under-cladding layer **11** may contain particles in addition to the above-mentioned resins. A sufficient S/N ratio can be obtained by dispersing the particles in the under-cladding layer. As the particles, any suitable particles capable of increasing the light reflectance of a surface of the under-cladding layer and/or reducing the light transparency in the under-cladding layer can be used. In the case where the particles are dispersed in the under-cladding layer, the light transmittance of the under-cladding layer at a wavelength of 650 nm may be, for example, 95% or less. When the under-cladding layer contains the particles, the accuracy required in optical coupling to the core layer can be reduced.

As a material for forming the particles, for example, there is given a metal or an inorganic oxide. In addition, an average particle diameter (ϕ) of the particles is, for example, from 10 nm to 5 µm. A filling ratio of the particles in the under-cladding layer is, for example, from 1% to 50%.

### B-2-3. Core Layer

The core layer **12** is formed into a substantially prism shape extending from the light incidence port **12a** side in a direction orthogonal to both an insertion and removal direction and a thickness direction of the SPR sensor cell, and in the illustrated example, is branched into two at a predetermined position in the above-mentioned direction. The core layer **12** is buried in the under-cladding layer **11** so that an upper surface thereof is exposed from the under-cladding layer **11.** The direction in which the core layer **12** extends is a waveguide direction of the optical waveguide.

The core layer **12** is arranged so that the upper surface thereof is flush with an upper surface of the under-cladding layer **11.** The metal layer can be arranged efficiently only on an upper side of the core layer by arranging the core layer so that the upper surface thereof is flush with the upper surface of the under-cladding layer. Further, the core layer is arranged so that both end surfaces thereof in the extending direction are flush with both end surfaces of the under-cladding layer in the extending direction, and the end surfaces function as the light incidence port **12a** and the light output port **12b,** respectively.

The core layer **12** preferably contains a halogen. When the core layer contains the halogen, the refractive index of the core layer can be decreased. As a result, the detection sensitivity can be enhanced remarkably. The SPR sensor cell according to this embodiment may have detection sensitivity sufficient for quantifying the amount of the object to be inspected (for example, an allergen) while realizing the downsizing that enables the use in a home environment. Examples of the halogen include fluorine, chlorine, bromine, and iodine. Fluorine is preferred. This is because it is easy to adjust the refractive index of the core layer to a desired refractive index. Further, the adhesion of the obj ect to be inspected (for example, an allergen) to the core layer can be suppressed through use of fluorine, and hence measurement accuracy can be increased.

As means for allowing the core layer to contain the halogen, any suitable means can be adopted. Specifically, it is appropriate that the core layer be formed through use of a halogen-containing material. As the halogen-containing material capable of forming a core layer, for example, there are given a halogen atom-containing resin and a halogen compound-containing resin composition. Specific examples of the halogen atom-containing resin include: fluorine atom-containing resins, such as polytetrafluoroethylene, a tetrafluoroethylene-hexafluoropropylene copolymer, a fluorinated epoxy resin, a fluorinated polyimide resin, a fluorinated polyamide resin, a fluorinated acrylic resin, a fluorinated polyurethane resin, and a fluorinated siloxane resin; chlorine atom-containing resins, such as a vinyl chloride resin, a vinyl chloride-ethylene copolymer, and a chlorinated polyolefin resin; and modified products thereof. A fluorine atom-containing resin is preferred. When the fluorine atom-containing resin is used, the refractive index of the core layer can be decreased to enhance sensitivity, and an ensuing decrease in S/N ratio can be suppressed. Further details are as follows. As described above, the refractive index of the core layer can be decreased to enhance sensitivity by using fluorine. On the other hand, when the refractive index of the core layer is decreased to enhance sensitivity, an SPR absorption peak is shifted to a long wavelength side (near-infrared region). In the near-infrared region, C-H vibration absorption is present, and light intensity at an excitation wavelength decreases due to the absorption. As a result, the S/N ratio may decrease or a waveguide mode may exert its influence. The vibration absorption can be shifted to a long wavelength side and a decrease in light intensity can be suppressed by bonding a fluorine atom heavier than a hydrogen atom to carbon, and hence the decrease in S/N ratio can be suppressed. Further, as described above, the adhesion of the object to be inspected (for example, an allergen) to the core layer can be suppressed through use of fluorine, and hence measurement accuracy can be increased. The fluorine atom-containing resin exemplified in the foregoing has a remarkable effect of suppressing the adhesion of the object to be inspected to the core layer. An example of the halogen compound-containing resin composition is a resin composition containing a halogen compound and an epoxy resin, a polyimide resin, a polyamide resin, a silicone resin, an acrylic resin, and/or a urethane resin. Specific examples of the halogen compound include hexabromobenzene, hexachlorobenzene, pentabromobenzene, pentachlorobenzene, pentabromophenol, pentachlorophenol, hexabromobiphenyl, decabromobiphenyl, chlorotetrabromobutane, tetrabromobutane, hexabromocyclododecane, perchloropentacyclodecane, decabromodiphenyl ether, octabromodiphenyl ether, hexabromodiphenyl ether, ethylenebis-tetrabromophthalimide, tetrachlorobisphenol A, tetrabromobisphenol A, brominated polystyrene, halogenated polycarbonate, a halogenated epoxy compound, brominated polyphenylene oxide, polychlorostyrene, chlorinated paraffin, tetrabromophthalic anhydride, and tetrachlorophthalic anhydride. The halogen-containing material (material for forming the core layer) may be used as a photosensitive material preferably by being blended with a photosensitizing agent.

The halogen content of the core layer **12** (substantially, the material for forming the core layer) is preferably 35 wt% or more, more preferably 40 wt% or more, still more preferably 50 wt% or more. When the halogen content falls within such range, a core layer having a desired refractive index is obtained, and as a result, there can be obtained an SPR sensor cell having detection sensitivity capable of realizing the quantification of the amount of an allergen in, for example, a home environment. Further, a core layer having a desired effect of suppressing the adhesion of the object to be inspected is obtained, and hence measurement accuracy can also be increased. On the other hand, the upper limit of the halogen content is preferably 78 wt%. When the upper limit is more than 78 wt%, the core layer may be liquefied or gasified and the shape of the core layer may not be maintained in some cases.

The refractive index of the core layer **12** is preferably 1.43 or less, more preferably 1.41 or less, still more preferably 1.39 or less. When the refractive index of the core layer is set to 1.43 or less, the detection sensitivity can be significantly increased, and the detection sensitivity capable of realizing the quantification of the amount of an allergen, for example, in a home environment can be realized. The lower limit of the refractive index of the core layer **12** is preferably 1.33. When the refractive index of the core layer **12** is 1.33 or more, SPR can be excited even in a sample of an aqueous solution system (refractive index of water: 1.33), and a general-purpose material can be used. The refractive index as used herein refers to a refractive index at a wavelength of 830 nm.

The refractive index of the core layer **12** is higher than that of the under-cladding layer **11.** The difference between the refractive index of the core layer and that of the under-cladding layer is preferably 0.010 or more, more preferably 0.020 or more. When the difference between the refractive index of the core layer and that of the under-cladding layer falls within such range, the optical waveguide of the detection unit can be set to a so-called multimode. Thus, the amount of light transmitted through the optical waveguide can be increased, and as a result, the S/N ratio can be increased. Further, when the optical waveguide is set to a multimode, various light is guided into the detection unit, and attenuation caused by SPR occurs in the various light. Therefore, the sensitivity can be increased.

The thickness of the core layer **12** is, for example, from 5 µm to 200 µm, preferably from 20 µm to 200 µm. In addition, the width of the core layer **12** is, for example, from 5 µm to 200 µm, preferably from 20 µm to 200 µm. When the core layer **12** has such thickness and/or width, the optical waveguide can be set to the so-called multimode.

### B-2-4. Metal Layer

As illustrated in FIG. **4** and FIG. **5****,** the metal layer **13** is formed so as to uniformly cover at least a part of the upper surface of the core layer **12.** As necessary, an easy-adhesion layer (not shown) may be formed between the core layer and the metal layer. By forming the easy-adhesion layer, the core layer can be fixed to the metal layer firmly.

As a material for forming the metal layer **13,** there are given gold, silver, platinum, copper, aluminum, and alloys thereof. The metal layer may be a single layer or may have a laminate structure of two or more layers. The thickness (total thickness of all the layers in the case of the laminate structure) of the metal layer is preferably from 20 nm to 70 nm, more preferably from 30 nm to 60 nm.

### B-2-5. Development Unit

As described above, in the SPR sensor cell, the development unit **15** extending across the plurality of optical waveguides (substantially, from the detection unit **13a** to the reference unit **13b**) may be arranged. The development unit **15** may be made of a porous material, for example, paper (for example, filter paper for paper chromatograph) or a porous film (for example, a non-woven fabric, a porous resin film). Through arrangement of the development unit, a liquid sample is enabled to uniformly reach the plurality of optical waveguides (substantially, the detection unit and the reference unit), and in this case, impurities, foreign matter, and the like in the sample can be removed. In the illustrated example, the development unit **15** is arranged across each entire width of the detection unit and the reference unit. However, for example, a trailing edge of the development unit **15** may be arranged in any suitable place of each surface of the detection unit and/or the reference unit within a range in which the above-mentioned effect can be exhibited. Further, unlike the illustrated example, the development unit may be arranged so as to cover each entire surface of the detection unit and the reference unit. The development unit may be arranged, for example, by fixing the above-mentioned material onto a desired place through use of an adhesive or the like.

The porous film has a pore diameter of preferably from 5 nm to 300 µm, more preferably from 10 nm to 100 µm, still more preferably from 25 nm to 50 µm, particularly preferably from 100 nm to 20 µm. When the pore diameter of the porous film is excessively small, there is a risk in that the object to be inspected may not pass or may not be transported through the porous film. When the pore diameter of the porous film is excessively large, there is a risk in that the foreign matter may not be removed. The porous film has a porosity of preferably from 25% to 95%, more preferably from 70% to 90%. Further, it is preferred that the protein adsorption ratio of the porous film be lower, and the protein adsorption ratio is preferably 300 µg/cm² or less, more preferably 100 µg/cm² or less, still more preferably 20 µg/cm² or less, particularly preferably 5 µg/cm² or less. When the protein adsorption ratio is excessively large, there is a risk in that the object to be inspected may adsorb to the porous film to make it impossible to perform measurement in the detection unit.

As the porous film, a porous resin film that is easily formed is typically used. Specific examples of a resin that may form the porous film include a fluorine-based resin (e.g., polytetrafluoroethylene: PTFE, or polyvinylidene fluoride: PVDF), an olefin-based resin (e.g., high-molecular-weight polyethylene, high-density polyethylene, low-density polyethylene, or polypropylene), a (meth)acrylic resin (e.g., polymethyl methacrylate), a styrene-based resin (e.g., polystyrene), a vinyl acetate-based resin (e.g., an ethylene-vinyl acetate copolymer), and a cellulose-based material (e.g., a cellulose-mixed ester). In particular, a material having low protein adsorption performance is preferred, and for example, a fluorine-based resin is preferred.

The porous film may be subjected to surface treatment as necessary. As a specific example of the surface treatment, there is given hydrophilic treatment.

The thickness of the development unit **15** is, for example, from 10 µm to 5 mm, preferably from 20 µm to 500 µm, more preferably from 30 µm to 100 µm, still more preferably from 50 µm to 90 µm. With such thickness, a liquid sample is enabled to satisfactorily reach both the detection unit and the reference unit, and in this case, impurities, foreign matter, and the like in the sample can be satisfactorily removed.

### B-2-6. Other Layers and Members

As a material for forming the easy-adhesion layer, there is typically given chromium or titanium. The thickness of the easy-adhesion layer is preferably from 1 nm to 5 nm.

As described above, a protective layer may be formed as necessary. The protective layer may be typically formed as a thin film having the same shape as that of the under-cladding layer in plan view so as to cover the entire upper surfaces of the under-cladding layer **11** and the core layer **12.** Through arrangement of the protective layer, when the sample is, for example, a liquid, the sample can be prevented from causing the core layer and/or the cladding layer to swell. As a material for forming the protective layer, there are given, for example, silicon dioxide and aluminum oxide. Those materials may be preferably adjusted so that the refractive index becomes lower than that of the core layer **12.** The thickness of the protective layer is preferably from 1 nm to 100 nm, more preferably from 5 nm to 20 nm.

The substrate **14** is a support substrate of the under-cladding layer. The substrate may be omitted as necessary. As a material for forming the substrate, any suitable resin may be used. Specific examples thereof include polyethylene terephthalate, polybutylene terephthalate, polyethylene naphthalate, polyethylene, polypropylene, polystyrene, and polyimide. The thickness of the substrate is, for example, from 50 µm to 2,000 µm, preferably from 100 µm to 500 µm.

### B-2-7. Anti-allergen Antibody

When the measurement device **200** is used for measuring (quantifying) the amount of an allergen, an anti-allergen antibody is fixed (typically, carried) onto the metal layer **13** to form the detection unit **13a** in the SPR sensor cell **100.** For example, when a mite allergen is quantified, an anti-mite allergen antibody is carried onto the metal layer. The procedure for carrying the anti-mite allergen antibody onto the metal layer is described below.
(1) A carboxyl group serving as a functional group is fixed onto the metal layer. Specifically, a solution that contains a thiol containing a carboxyl group (for example, 7-carboxy-1-heptanethiol) and the metal layer are brought into contact with each other for a predetermined time period. The contact may be typically performed by dropping of the solution or immersion into the solution. The contact time is, for example, from 20 minutes to 720 minutes, preferably about 60 minutes. The solution has a concentration of, for example, 60 mM.
(2) The metal layer is washed with ultrapure water to remove an unfixed thiol compound.
(3) A solution containing a water-soluble carbodiimide and hydroxysuccinimide and the metal layer are brought into contact with each other. The contact may be typically performed by dropping of the solution or immersion into the solution. With this, the carboxyl group fixed onto the metal layer can be activated. This solution may contain, for example, the carbodiimide and hydroxysuccinimide in a molar ratio of 1:1.
(4) The metal layer is washed with water to remove the solution adhering to the metal layer.
(5) An antibody solution in which an anti-mite allergen antibody is dissolved or dispersed in a buffer solution and the metal layer are brought into contact with each other for a predetermined time period. With this, the activated carboxyl group fixed onto the metal layer and the anti-mite allergen antibody bind to each other, with the result that the anti-mite allergen antibody is carried onto the metal layer. The concentration of the anti-mite allergen antibody in the antibody solution maybe set to, for example, 100 µg/ml. The contact time may be set to, for example, about 60 minutes.
(6) Ethanol containing an amino group (for example, 10 mM ethanolamine) and the metal layer are brought into contact with each other, to thereby block the carboxyl group that has been fixed on the metal layer, but not bound to the anti-mite allergen antibody.

Through the above-mentioned operations (1) to (6), the anti-mite allergen antibody that performs an antigen-antibody reaction with the mite allergen (antigen) is carried onto the metal layer. In addition to the above-mentioned procedure, for example, a method disclosed in JP 2003-156434 A, JP 2009-216483 A, WO 90/5303 A1, US 5716854 A, US 5922594 A, or US 8012587 B2 may also be used.

The anti-mite allergen antibody is a protein that specifically binds to the mite allergen (antigen) that causes mite allergy. As the mite allergen (antigen), Derf I or Derf II which is an allergy antigenic substance of Dermatophagoides farinae, and Derp I or Derp II which is an allergy antigenic substance of Dermatophagoides pteronyssinus can be targeted. Der I (Derf I and Derp I) is a protein having a molecular weight of about 25K, which has cysteine protease activity and is frequently found in excrements of mites. Therefore, it is considered that Der I may be a gastrointestinal enzyme. Der II (Derf II and Derp II) is a protein having a molecular weight of about 14K and is frequently found in mite bodies. The amino acid sequences of Derf I, Derf II, Derp I, and Derp II are well-known from literatures and the like. Corresponding allergens of Dermatophagoides farinae and Dermatophagoides pteronyssinus have significantly high structural similarity. For example, the amino acid sequences are 78% and 88% identical between Derf I and Derp I and between Derf II and Derp II, respectively. In particular, as an indicator of the amount of mite allergens, a Der I amount (total amount of Derf I and Derp I: amount of group 1 mite allergens) may be used. Specifically, a Der I amount of 2 µg/g in house dust may be defined as a threshold value of sensitization, and a Der I amount of 10 µg/g may be defined as a threshold value of induction of an attack of asthma.

### B-3. Sample Mounting Portion

The sample mounting portion **20** is defined by an upper surface of the optical waveguide main body **10** and an over-cladding layer **21.** Specifically, as illustrated in FIG. **3** to FIG. **5****,** the over-cladding layer **21** is formed into a rectangular frame shape in plan view so that, in the upper surface of the optical waveguide main body **10,** an outer periphery of the over-cladding layer **21** is substantially the same as that of the optical waveguide main body **10** in plan view, and a portion surrounded by the over-cladding layer **21** and the upper surface of the optical waveguide main body **10** is defined as the sample mounting portion **20.** When a sample is mounted in this compartment, the metal layer (substantially, the recognition substance fixed onto the metal layer) and the sample (substantially, the object to be inspected in the sample) are brought into contact with each other, to thereby enable the detection. Further, the sample can be easily mounted on the surface of the metal layer by forming such compartment, and hence the operability can be enhanced. It is only necessary that the width of the sample mounting portion be a width which enables the sample to move to the metal layer through a capillary phenomenon or the like.

As a material for forming the over-cladding layer **21,** there are given, for example, the materials for forming the core layer and the under-cladding layer, silicone rubber, a resin film, and an inorganic oxide film. The material for forming the over-cladding layer may be a pressure-sensitive adhesive tape including a base film and a pressure-sensitive adhesive layer. The thickness of the over-cladding layer is preferably from 5 µm to 1,000 µm, more preferably from 25 µm to 200 µm. The refractive index of the over-cladding layer is preferably lower than that of the core layer. In one embodiment, the refractive index of the over-cladding layer is equal to that of the under-cladding layer.

The cell main body **110** can be manufactured by any suitable method. As a specific example of the manufacturing method, there is given a method described in JP 2012-215540 A.

### B-4. Holder

The holder **120** includes an upper plate **30** and a lower plate **40,** and the cell main body **110** is held by the upper plate **30** and the lower plate **40.**

As illustrated in FIG. **5** to FIG. **7****,** the lower plate **40** has an upper surface in which a cell main body mounting portion **41** is formed to be recessed so that the cell main body **110** can be fitted therein. Meanwhile, the upper plate **30** has a bottom surface in which a push-in portion **31** that has a shape corresponding to the cell main body mounting portion **41** in plan view and protrudes downwardly is formed. The depth of the cell main body mounting portion **41** is set to be larger than the thickness of the push-in portion **31** by the thickness of the cell main body **110** (total of the thickness of the optical waveguide main body and the thickness of the over-cladding layer). Thus, the cell main body can be held and fixed by mounting the cell main body **110** on the cell main body mounting portion **41** of the lower plate **40** and pushing the upper plate **30** from above the cell main body **110** so that the push-in portion **31** is fitted into the cell main body mounting portion **41.**

In this embodiment, the upper plate **30** and the lower plate **40** include engagement portions capable of being engaged with each other, and the cell main body is strongly held and fixed through engagement of those engagement portions. Specifically, as illustrated in FIG. **5** to FIG. **7****,** the lower plate **40** has a side wall **42** standing from a peripheral edge of the upper surface thereof, and a plurality of engagement claws **43** each having a downward-pointing hook shape, which protrude toward an inner side, are formed at an upper end of the side wall **42.** The height of the side wall **42** is substantially the same as the thickness of the upper plate **30** (thickness of a region in which the push-in portion is not formed). Meanwhile, the upper plate **30** includes engagement grooves **32** each having a shape corresponding to the engagement craw **43** along a peripheral edge of the upper surface of the upper plate **30.** As described above, the upper plate **30** is pushed in to engage the engagement claws **43** and the engagement grooves **32** with each other, with the result that the cell main body **110** can be easily and strongly held and fixed. Openings **45** are formed at positions of the side wall **42** corresponding to the light incidence port **12a** and the light output ports **12b.** Holding and fixing of the cell main body are not limited to the engagement of the upper plate and the lower plate, and the cell main body may be held and fixed through use of, for example, screws, an adhesive, or the like.

In the upper plate **30,** a first through hole **33** and a second through hole **34,** each passing through the upper plate **30** in a vertical direction, are formed on both sides of the two metal layers **13** in plan view, respectively, that is, on the cell insertion side and the measurement device main body side with the two metal layers **13** interposed therebetween. When the upper plate **30** and the lower plate **40** are integrated as the holder **120** so as to hold and fix the cell main body **110,** the first through hole **33** and the second through hole **34** each communicate to the sample mounting portion **20.** In this configuration, the first through hole **33** is used as a sample introduction portion, and the second through hole **34** serves as a ventilation hole. Therefore, the sample having been introduced into the first through hole **33** flows through the sample mounting portion **20** in the direction of the measurement device main body to be brought into contact with the metal layer **13** through the capillary phenomenon, to thereby enable measurement. The first through hole **33** is formed at a position that is to be placed on an outer side of the measurement device main body when the SPR sensor cell **100** is mounted on the cell mounting portion. With such configuration, the sample can be introduced after the SPR cell sensor cell is mounted on the cell mounting portion, and hence operability can be enhanced.

The inner diameter of the first through hole **33** is typically from 2 mm to 10 mm, and about 10 µl of the sample can be introduced into the first through hole **33.** Further, the inner diameter of the second through hole **34** is typically from 0.1 mm to 2 mm.

As materials for forming the upper plate **30** and the lower plate **40,** any suitable materials may be used depending on the purpose and the like. Specific examples of the formation materials include polyethylene terephthalate, polybutylene terephthalate, polyethylene naphthalate, polyethylene, polypropylene, polystyrene, and polyimide. The thickness of the upper plate **30** (thickness of a portion in which the push-in portion is not formed) is, for example, from 0.5 mm to 5 mm, preferably from 0.8 mm to 3 mm. The thickness of the lower plate **40** (thickness of a portion in which the cell main body mounting portion is not formed) is, for example, from 0.5 mm to 8 mm, preferably from 0.5 mm to 5 mm.

Each of the upper plate **30** and the lower plate **40** may be manufactured by any suitable method, for example, injection molding or cutting.

### C. Method of Using Measurement Device

Now, as an example of a method of using the measurement device, a quantification method for a mite allergen is described. First, in a room in a house, a building, or the like to be inspected for mite contamination, dust is collected, for example, with a cleaning tool that includes a filter having filter openings capable of collecting the dust. The dust may be collected by wiping the dust with the cleaning tool or by sucking the dust with a vacuum cleaner when the cleaning tool is the vacuum cleaner. For example, when the dust is sucked with the vacuum cleaner, a mite allergen in the room is sucked into the vacuum cleaner through the suction and collected into the filter. The filter is removed from the vacuum cleaner, and a predetermined amount of the dust collected with the filter is dissolved or dispersed in a predetermined amount of a buffer solution (for example, a Tris-buffered solution, TBS, MOPS, a phosphate buffer solution) to prepare a sample liquid. Further, for example, in the case of a cyclone-type vacuum cleaner, a sample liquid may be prepared by pouring a buffer solution to the dust collected into a collecting container or transferring the collected dust to another container and pouring the buffer solution to the dust. Meanwhile, as illustrated in FIG. **1****,** the SPR sensor cell is mounted on the measurement device main body, and the sample liquid is dropped from the sample introduction portion (first through hole) . Then, light is caused to enter the core layer from the light source after an elapse of a predetermined time period. As a result, surface plasmon resonance occurs to change light intensity, and the amount of the mite allergen can be quantified based on the light intensity change amount. In the case of the measurement device using the SPR sensor cell as illustrated in FIG. **3** to FIG. **5****,** a light source noise, an environmental noise, and the like can be determined based on a change in light intensity of the reference unit **13b.** Therefore, the amount of the mite allergen can be measured (quantified) more precisely by correcting a change in light intensity of the detection unit **13a** with the change in light intensity of the reference unit **13b.** Further, when the number of the optical waveguides is increased, all the amounts of Derf I, Derf II, Derp I, and Derp II can be quantified significantly precisely as described above. As a result, a relationship between the amount of the mite allergen in house dust, and the threshold value of sensitization and/or the threshold value of induction of an attack of asthma can be determined. A series of those operations can be performed even in a home environment, and can be performed even by a person (for example, a housewife) having no expertise. As a result, the mite allergen in a home environment can be found early and quantified. Therefore, the measurement device according to the embodiment of the present invention is significantly effective for prevention of allergy diseases caused by mites. Specifically, when appropriate indoor cleaning is performed while the amount of the mite allergen is measured periodically (for example, every week) and continuously (for example, about one year), the mite allergen in a room and house dust can be significantly reduced. As a result, the effect of prevention of allergy diseases caused by mites can be significantly improved.

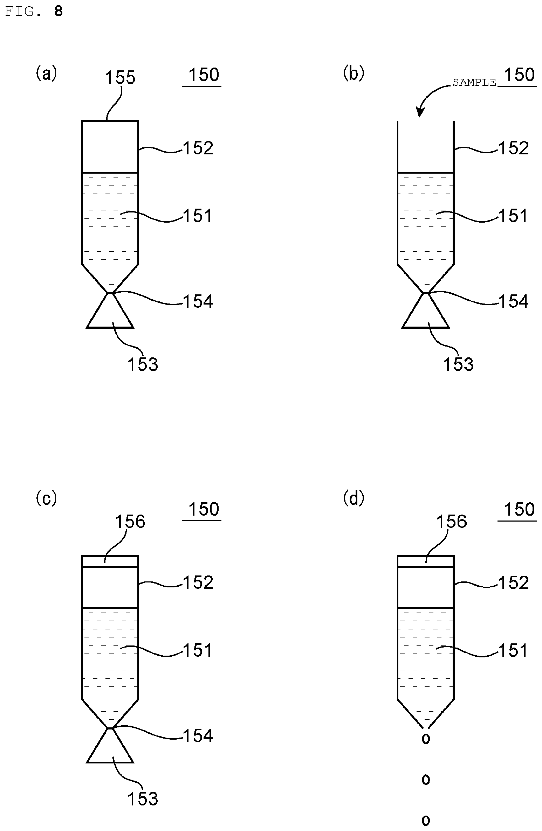

As described above, the measurement device according to the embodiment of the present invention can be used by a person (for example, a housewife) having no expertise in a home environment. From such viewpoint, the measurement device may be provided as a set including predetermined members. Specifically, the measurement device set may include: the measurement device main body having the SPR sensor cell mounted thereon, which can be used continuously by replacing the cell with a new cell; the SPR sensor cell that is used by being replaced for each measurement of the amount of an allergen; and a sample liquid preparation and dropping member capable of preparing a sample liquid from collected house dust and dropping the sample liquid onto the SPR sensor cell. The SPR sensor cell and the measurement device main body are as described above in the sections A and B. The sample liquid preparation and dropping member is configured, for example, as follows. The sample liquid preparation and dropping member is filled with a predetermined amount of the above-mentioned buffer solution and sealed before use. At the time of use, house dust can be supplied to the buffer solution to be dissolved or dispersed therein to prepare a sample liquid. Further, the sample liquid can be dropped from the member onto the sample introduction portion of the SPR sensor cell. Such sample liquid preparation and dropping member does not need to measure the buffer solution and needs to measure only the house dust. Therefore, the operation of the sample liquid preparation and dropping member is simple, and the concentration of the house dust (as a result, mite allergen) in the sample liquid can be adjusted more accurately. Specifically, as illustrated in FIG. **8(a)**, the member includes : a main body **152** which has a tapered tip end portion and is filled with a buffer solution **151;** a first sealing portion **154** which is arranged in the tapered tip end portion and which is sealed before use and opened by breaking off a folding portion **153** at the time of use; and a second sealing portion **155** which is arranged in a main body end portion on an opposite side of the first sealing portion **154,** is sealed with a sealing material before use, the sealing material being peelably bonded thereto, and is capable of being supplied with a sample (house dust) by peeling the sealing material at the time of use. At the time of use, first, as illustrated in FIG. **8(b)****,** the sealing material is peeled from the second sealing portion **155,** and a predetermined amount of a sample (house dust), which has been collected and measured, is supplied from a portion opened by the peeling to be dispersed or dissolved in the buffer solution, with the result that a sample liquid having a predetermined concentration is prepared. Then, as illustrated in FIG. **8(c)****,** the portion from which the sealing material has been peeled is closed with a lid **156** so that the sample liquid does not leak. Finally, as illustrated in FIG. **8(d)****,** the folding portion **153** is broken off, and the sample liquid is dropped from a portion opened by breaking off the folding portion **153** onto the measurement device (substantially, the sample introduction portion of the SPR sensor cell). Such sample liquid preparation and dropping member may be preferably made of a transparent resin. The reasons for this are as follows. The leakage of the buffer solution and/or the sample liquid caused by cracking can be prevented. Further, the sample liquid preparation and dropping member can be deformed with a pressing force and an inner portion thereof can be visually recognized. Therefore, a dropping operation is easy. The concentration of the sample liquid (concentration of dust, concentration of house dust) in quantification of the amount of an allergen using the measurement device according to the embodiment of the present invention is, for example, from 10 µg/ml to 10 g/ml, preferably from 100 µg/ml to 1 g/ml, more preferably from 1 mg/ml to 500 mg/ml.

As means for collecting dust, a wiping member may be used. Thus, the above-mentioned set may include the wiping member. In one embodiment, the wiping member is a sheet-like or flat bar-like member including a roughened or porous collecting portion. In this case, a measurer (for example, a housewife) wipes (or rubs) a test object with the wiping member to collect a sample, and prepares and drops a sample liquid through use of the sample liquid preparation and dropping member, thereby being able to quantify the amount of a mite allergen. Further, in another embodiment, a sheet having a sample collected thereon is immersed in the above-mentioned buffer solution to extract the allergen, to thereby prepare a sample liquid (extraction liquid), and the extraction liquid is dropped onto the sample introduction portion of the SPR sensor cell, with the result that the amount of the mite allergen can be quantified. Further, in another embodiment, the SPR sensor cell can be used for collecting a sample. For example, a sample can be collected through use of the SPR sensor cell by imparting a collecting (typically, wiping) function to the upper plate of the SPR sensor cell. More specifically, the first through hole (sample introduction portion) of the upper plate as illustrated in FIG. **5** is caused to protrude upwardly, and a porous collecting portion is arranged in an (upper) end portion of the protruding portion. The collection of a sample may be performed under a state in which the cell main body is fixed with the upper plate (and the lower plate). Alternatively, after the collection of a sample is performed only with the upper plate, the upper plate may be mounted and fixed onto the cell main body. An extraction liquid (for example, the above-mentioned buffer solution) is dropped onto the collecting portion of the SPR sensor cell including the upper plate having the sample collected thereon and caused to pass through the porous collecting portion, to thereby extract a sample liquid. The extracted sample liquid is dropped to reach the sample mounting portion. Thus, the amount of the mite allergen can be quantified through use of the sample collected with the SPR sensor cell.

When the quantification of the amount of the mite allergen is performed through use of the measurement device according to the embodiment of the present invention in a home environment, it is preferred that the quantification be performed periodically and continuously in a predetermined point (preferably a plurality of points). Through use of such quantification, a more appropriate cleaning method (removal method for the mite allergen) can be clarified, and the amount of the mite allergen can be significantly reduced by periodically and continuously performing the quantification and the appropriate cleaning method in combination. As a result, allergy diseases caused by mites can be significantly effectively prevented.

### Examples

The present invention is hereinafter described specifically by way of Examples. However, the present invention is not limited to these Examples.

### [Example 1]

In this Example, the effect of providing a reference unit and a development unit was verified.

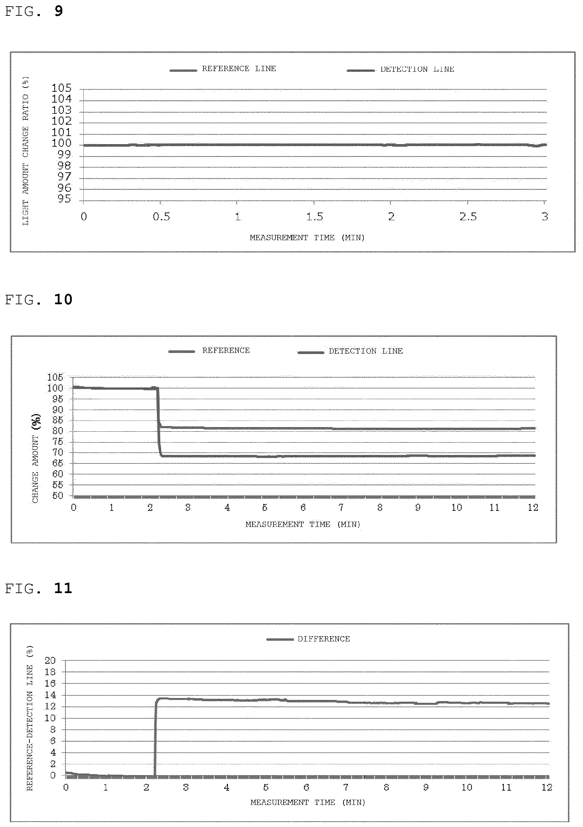

First, the SPR sensor cell as illustrated in FIG. **3** to FIG. **5** was manufactured by a method in accordance with a method described in Example 1 of JP 2012-215540A. An optical waveguide (substantially, a core layer) was branched into two optical waveguides. An antibody against Derf I was carried onto a metal layer on one of the core layers to form a detection unit, and a metal layer on the other core layer was used as it is as a reference unit. Further, a development unit (membrane filter made of hydrophilic polytetrafluoroethylene (PTFE) (Omnipore, JAWP09025, manufactured by Merck Ltd.) having a pore diameter of 1.0 µm, a thickness of 85 µm, a porosity of 80%, and a protein adsorption ratio of 4 µg/cm²) was arranged on the optical waveguides (substantially, across the two metal layers). The SPR sensor cell was inserted to be mounted on the cell mounting portion of the measurement device main body, to thereby provide the measurement device as illustrated in FIG. **1** and FIG. **2****.** A transmittance was measured for 3 minutes immediately after mounting of the SPR sensor cell. It was confirmed that the value thereof was substantially constant (FIG. **9**), and the value was defined as a reference value (100%).

Next, dust was collected from a carpet in a room, and a sample liquid was prepared through use of the member as illustrated in FIGS. **8****.** The sample liquid was dropped onto the sample introduction portion of the SPR sensor cell mounted on the measurement device main body. A transmittance in each of the detection unit and the reference unit was measured for 12 minutes immediately after dropping. As a result, the transmittance changed in any of the detection unit and the reference unit within about two minutes from dropping of the sample liquid, and a change ratio in the detection unit was significantly larger than that in the reference unit. FIG. **10** is a graph for showing the comparison of a transmittance change between the detection unit and the reference unit, and FIG. **11** is a graph for showing the difference in transmittance change between the detection unit and the reference unit.

As is apparent from FIG. **10** and FIG. **11****,** according to this Example of the present invention, a light source noise and an environmental noise caused by the influence of a non-specific substance can be eliminated through the correction using the transmittance change in the reference unit (that is, calculating the difference), and hence a change caused by the allergen in the detection unit can be determined with high sensitivity and high accuracy.

### [Example 2]

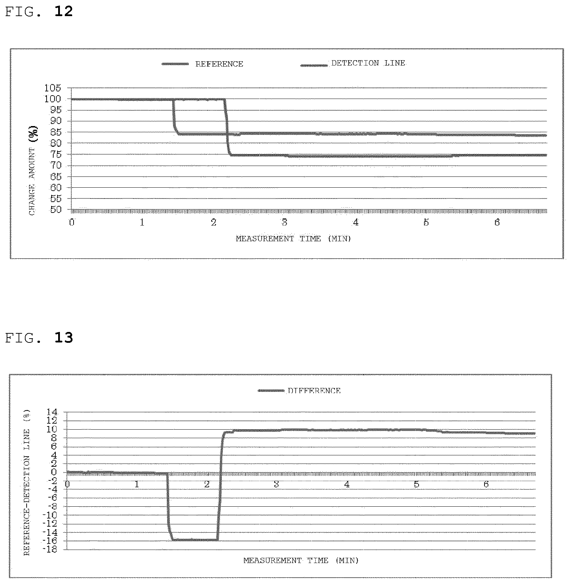

An SPR sensor cell was manufactured in the same manner as in Example 1 except that the development unit was not provided. FIG. **12** is a graph for showing the comparison of a transmittance change between the detection unit and the reference unit, and FIG. **13** is a graph for showing the difference in transmittance change between the detection unit and the reference unit. As shown in FIG. **12** and FIG. **13****,** a change ratio in the detection unit was larger than that in the reference unit after an elapse of about two minutes from dropping of the sample liquid and was able to be measured and quantified with allowable sensitivity and accuracy. However, as shown in FIG. **12** and FIG. **13****,** a transmittance in only the reference unit changed within about one minute from dropping of the sample liquid, and hence a reverse phenomenon occurred in the difference in transmittance change between the detection unit and the reference unit within this time range. It is understood from the foregoing that, through arrangement of the development unit as in Example 1, the sample liquid is allowed to uniformly permeate the optical waveguides (more specifically, the detection unit and the reference unit), and a more accurate reaction between the sample liquid (obj ect to be inspected) and the recognition substance is enabled. As a result, it is understood that, through arrangement of the development unit as in Example 1, a change caused by the allergen in the detection unit can be determined with higher sensitivity and higher accuracy.

### [Comparative Example 1]

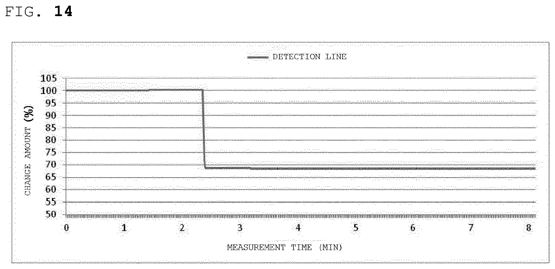

An SPR sensor cell was manufactured in the same manner as in Example 1 except that none of the reference unit and the development unit was provided. FIG. **14** is a graph for showing a transmittance change in the detection unit. The reference unit was not provided, and hence a light source noise and an environmental noise caused by the influence of a non-specific substance were not able to be corrected, and it was necessary that measurement and quantification be performed only based on the transmittance change in the detection unit.

### Industrial Applicability

The measurement device of the present invention may be used in general measurement of the amount of an obj ect to be inspected, which may use the SPR phenomenon, and may be used suitably, in particular, for quantification of an allergen (typically a mite allergen).

### Reference Signs List

- **10**: optical waveguide main body

- **20**: sample mounting portion
- **30**: upper plate
- **40**: lower plate
- **100**: SPR sensor cell
- **110**: cell main body
- **120**: holder
- **200**: measurement device
- **210**: cell mounting portion
- **220**: light source
- **230**: light receiving element
- **240**: optical fiber

## Claims

1. A measurement device, comprising:
an SPR sensor cell;
a light source; and
a light receiving element,
wherein the SPR sensor cell includes a plurality of optical waveguides, at least one of the plurality of optical waveguides forming a detection unit together with a metal layer onto which a recognition substance for an object to be inspected is fixed, at least another one of the plurality of optical waveguides forming a reference unit together with a metal layer free of the recognition substance.

2. The measurement device according to claim 1, further comprising a development unit extending across the plurality of optical waveguides.

3. The measurement device according to claim 2, wherein the development unit comprises a porous material.

4. The measurement device according to any one of claims 1 to 3, wherein the detection unit includes an under-cladding layer, a core layer formed so that at least a part thereof is adjacent to the under-cladding layer, and a metal layer configured to cover the core layer, and the recognition substance is fixed onto the metal layer.

5. The measurement device according to any one of claims 1 to 4, wherein the SPR sensor cell is removably mounted.

6. The measurement device according to any one of claims 1 to 5, wherein the recognition substance comprises an anti-allergen antibody, and the measurement device is configured to measure an amount of an allergen by an antigen-antibody reaction with the anti-allergen antibody.
